# EUROPEAN PATENT SPECIFICATION

(11) **EP 3 906 957 B1**
(45) Date of publication and mention of the grant of the patent: **21.05.2025**
(21) Application number: 21170149.5
(22) Date of filing: 16.03.2016
(51) Int. Cl.: A61M 16/06, A61M 16/00, A61M 16/16

(54) **PATIENT INTERFACE**
PATIENTENSCHNITTSTELLE
INTERFACE PATIENT

(30) Priority: 16.03.2015 US 201562133908 P; 23.04.2015 US 201562151898 P; 09.11.2015 US 201562252943 P
(43) Date of publication of application: 10.11.2021
(62) Divisional of application: 16765326.0
(73) Proprietor: Fisher & Paykel Healthcare Limited, East Tamaki Auckland 2013 (NZ)
(72) Inventor: RONAYNE, Michael Paul, 2013 Auckland (NZ); JOHNSON, Chelsea Erin, 2013 Auckland (NZ); MILNE, Robert Andrew David, 2013 Auckland (NZ)
(74) Representative: Treeby, Philip David William

(56) References cited:
- WO-A1-2012/104756
- US-A1- 2006 060 200
- US-A1- 2010 229 872

## Description

### BACKGROUND

### Technical Field

The present disclosure generally relates to respiratory therapy. More particularly, the present disclosure relates to a patient interface for providing respiratory therapy.

### Description of the Related Art

A patient dealing with respiratory illness, for example chronic obstructive pulmonary disease (COPD), can have difficulty engaging in effective respiration. This difficulty may be the result of a variety of causes, including a breakdown of lung tissue, dysfunctions of the small airways, excessive accumulation of sputum, infection, genetic disorders, or cardiac insufficiency. With some respiratory illnesses, it is useful to provide a patient with a therapy that can improve the ventilation of the patient. The patient can be provided with high flow therapy using a respiratory therapy system that includes a gases source, a patient interface that may be used to transmit gases to an airway of a patient, and a conduit extending between the gases source and the patient interface. The patient interface is typically not sealed. The gases may be heated and humidified before delivery to the patient.

Obstructive Sleep Apnea (OSA) is a sleep disorder in which muscles that normally hold open the airway collapse, temporarily sealing the airway. The sleep pattern of an OSA sufferer is characterized by repeated sequences of snoring, breathing difficulty, lack of breathing, waking with a start, and then returning to sleep. The respiratory therapy of Continuous Positive Airway Pressure (CPAP) can be used to splint the airway and reduce or eliminate the occurrence of OSA. The patient can be provided with CPAP therapy using a respiratory therapy system that includes a gases source, a patient interface that may be used to transmit gases to an airway of a patient, and a conduit extending between the gases source and the patient interface. The patient interface is typically sealed. The gases may be heated and humidified before delivery to the patient.

US 2006/060200 A1 discloses a patient interface that includes a support body having a cushion and cheek interfaces. Collectively, the cushion and cheek interfaces operate to distribute compressive forces exerted on the user's face. The support body of the patient interface is constructed from a flexible material to impart additional adjustability to the cheek interfaces. The support body also has arcuate eyelets designed to provide a hinge between the headgear assembly and the support body.

### SUMMARY

According to the present invention there is provided a fixation structure as defined in claim 1.

Patient interfaces for respiratory therapy systems can include nasal cannulas, nasal masks, oral masks, oro-nasal masks, full face masks, nasal pillows masks, or endotracheal tubes. When a patient interface is fitted to a patient, gases are channeled through nasal delivery elements of the patient interface to the patient's airway (for example, the tubular prongs of a nasal cannula, which rest in the nares of the patient). In some cases, it is useful to attach the patient interface to the patient's face. One option is to position an adhesive pad on the patient's face, the adhesive pad having a mechanical fastener on a side of the adhesive pad that faces away from the patient's face. The mechanical fastener couples with a complementary fastener attached to the patient interface. However, depending on factors including the shape of the adhesive pad, separation of the mechanical fastener from the complementary fastener can cause the adhesive pad to be undesirably dislodged from the patient's face. Solutions to the above difficulties, or systems or apparatus that provide a useful alternative, are sought.

In accordance with certain features, aspects and advantages of one or more of the embodiments disclosed herein, a fixation structure is disclosed. The fixation structure is adapted to cooperate with a patient interface to secure the patient interface on a face of a patient. The fixation structure comprises a first edge adapted to face toward a nose or mouth of the patient in use. The fixation structure comprises a second edge adapted to face away from the nose or mouth of the patient in use. The first edge is substantially flat or straight. The first and second edges are substantially the same width.

The first and second edges can be identical in shape and size. The fixation structure can comprise a substantially flat or straight third edge extending between the first and second edges. The fixation structure can comprise substantially flat or straight third and fourth edges extending between the first and second edges. The second edge can be substantially rounded. The fixation structure can comprise a periphery with a substantially triangular shape. The fixation structure can comprise a periphery with a substantially curved shape.

The fixation structure can comprise a first fastener adapted to interface with a complementary second fastener of an attachment structure of the patient interface. The first fastener can be secured or adhered to a first surface of the fixation structure that faces away from the patient's face in use. The first fastener can cover substantially the entire first surface of the fixation structure.

The fixation structure can comprise opposing third and fourth edges extending between the first and second edges. The first fastener can extend substantially along the entirety of the first surface of the fixation structure between the first and second edges and only partly between the opposed third and fourth edges.

The first fastener can extend from the first edge to a position along the length of the fixation structure between the first and second edges. The fixation structure can comprise opposing third and fourth edges extending between the first and second edges. The first fastener can extend from the first edge to a position along the length of the fixation structure between the first and second edges. The first fastener can extend from the third edge to a position along the width of the fixation structure between the third and fourth edges.

The first fastener can comprise a hooked fastener adapted to interface with a complementary looped fastener of the attachment structure of the patient interface. The hooked fastener can comprise hooks that extend outwardly from a side of the fixation structure that faces away from the patient's face. The hooks can extend toward the first and/or second edges of the fixation structure.

The hooks can be generally parallel with the first and second edges and generally perpendicular with the third and fourth edges when the third edge is substantially flat.

The fixation structure can be of substantially uniform thickness.

The fixation structure can be constructed from a hydrocolloid-based adhesive. The fixation structure can be constructed from a silicone-based adhesive.

The fixation structure can be adapted to facilitate connecting the fixation structure to the face of the patient. For example, the patient-facing region of the fixation structure can be constructed from a material that in use substantially maintains a constant level of adherence to the skin for at least a predetermined period of time after application to the skin.

Additionally, in accordance with certain features, aspects and advantages of one or more of the embodiments disclosed herein, a fixation structure is disclosed. The fixation structure is adapted to cooperate with a patient interface to secure the patient interface on a face of a patient. The fixation structure comprises a body with a patient-facing region adapted to adhere to the face of the patient. The fixation structure comprises a first fastener adhered to the body. The first fastener is adapted to interface with a complementary second fastener of an attachment structure of the patient interface. The body is constructed from a hydrocolloid-based adhesive or a silicone-based adhesive.

Additionally, in accordance with certain features, aspects and advantages of one or more of the embodiments disclosed herein, a fixation structure is disclosed. The fixation structure is adapted to cooperate with a patient interface to secure the patient interface on a face of a patient. The fixation structure comprises a first edge adapted to face toward a nose or mouth of the patient in use. The fixation structure comprises a second edge adapted to face away from the nose or mouth of the patient in use. The fixation structure comprises a hooked fastener adapted to interface with a complementary looped fastener of an attachment structure of the patient interface. The hooked fastener extends at least partially between the first and second edges. The hooked fastener comprises hooks that extend toward the first and/or second edges of the fixation structure.

Additionally, in accordance with certain features, aspects and advantages of one or more of the embodiments disclosed herein, a fixation structure is disclosed. The fixation structure is adapted to cooperate with a patient interface to secure the patient interface on a face of a patient. The fixation structure comprises a first edge adapted to face toward a nose or mouth of the patient in use. The fixation structure comprises a second edge adapted to face away from the nose or mouth of the patient in use. The fixation structure comprises a third edge extending between the first and second edges. The third edge comprises a concave curve.

The fixation structure can comprise a fourth edge extending between the first and second edges. The fourth edge can comprise a concave curve that is shaped to match a shape of an under-eye region of the patient. The third and fourth edges can be opposed.

The fixation structure can comprise a first fastener adapted to interface with a complementary second fastener of an attachment structure of the patient interface. The first fastener can be fixed to a side of the fixation structure that faces away from the patient. A midpoint of the first fastener can be offset from a first centerline or central portion that is midway between the first and second edges of the fixation structure. The first fastener can be positioned such that it is closer to the first edge than to the second edge. The midpoint of the first fastener can be on a second centerline or central portion that is midway between the third and fourth edges of the fixation structure.

Additionally, in accordance with certain features, aspects and advantages of one or more of the embodiments disclosed herein, a fixation structure is disclosed. The fixation structure is adapted to cooperate with a patient interface to secure the patient interface on a face of a patient. The fixation structure comprises a first edge adapted to face toward a nose or mouth of the patient in use. The fixation structure comprises a second edge adapted to face away from the nose or mouth of the patient in use. The fixation structure comprises a third edge extending between the first and second edges. The third edge projects farther from a centerline or center portion of the fixation structure at portions of the third edge proximal to the first and second edges than at portions of the third edge distal from the first and second edges.

The portions of the third edge proximal to the second edge can project farther from the centerline or central portion of the fixation structure than portions of the third edge proximal to the first edge.

Additionally, in accordance with certain features, aspects and advantages of one or more of the embodiments disclosed herein, a fixation structure is disclosed. The fixation structure is adapted to cooperate with a patient interface to secure the patient interface on the face of the patient. The fixation structure comprises a body adapted to adhere to the face of the patient. The body is constructed at least in part from a material having a low hygroscopicity.

Additionally, in accordance with certain features, aspects and advantages of one or more of the embodiments disclosed herein, a fixation structure is disclosed. The fixation structure is adapted to cooperate with a patient interface to secure the patient interface on the face of the patient. The fixation structure comprises a body adapted to adhere to the face of the patient. The body is constructed at least in part from a material having a low Young's modulus.

The body can have a thickness that allows the body to flex in response to movements of the patient's face. The body can have a thickness that is between 0.05 and 5.0 mm. The thickness of the first fastener can be less than the thickness of the body. The thickness of the first fastener can be at least a quarter of the thickness of the body.

The body can comprise a silicone-based adhesive that conforms to the shape of the patient's skin in use. The body can comprise a hydrocolloid-based adhesive.

Additionally, in accordance with certain features, aspects and advantages of one or more of the embodiments disclosed herein, a fixation structure is disclosed. The fixation structure cooperates with a patient interface to secure the patient interface on the patient's face. The fixation structure comprises a body that adheres to the patient's face in use. The body comprises a first fastener that couples with a complementary second fastener of an attachment structure of the patient interface. The body comprises an adhesive that adheres to the skin on the patient's face in use. The adhesive substantially immediately reaches a maximum amount of adherence to the skin upon application of the adhesive to the skin.

The adhesive can substantially immediately flow into gaps in the skin upon application of the adhesive to the skin. The adhesive can be a silicone-based adhesive. The adhesive can require less force to remove than a similarly sized hydrocolloid-based adhesive applied to the skin of the patient's face.

Additionally, in accordance with certain features, aspects and advantages of one or more of the embodiments disclosed herein, a fixation structure is disclosed. The fixation structure cooperates with a patient interface to secure the patient interface on the patient's face. The fixation structure comprises a first edge that faces toward a patient's nose or mouth in use. The fixation structure comprises a second edge that faces away from the patient's nose or mouth in use. The fixation structure comprises a plurality of slits that facilitate flexibility of the fixation structure.

Additionally, in accordance with certain features, aspects and advantages of one or more of the embodiments disclosed herein, a patient interface system is disclosed. The patient interface system comprises a patient interface comprising an attachment structure secured to a patient-facing portion of the patient interface. The attachment structure couples with a fixation structure secured to the face to fasten the patient interface to the face in use. The fixation structure may be the same or similar to any of the fixation structures described in the passages above or elsewhere in the specification with reference to the accompanying figures.

The patient interface can comprise a first body adapted to rest on a first portion of the patient's face, a second body adapted to rest on a second portion of the patient's face, and a bridge linking the first and second bodies.

The attachment structure can be secured to patient-facing regions of the first and/or second bodies.

The patient interface can comprise a nasal cannula, nasal mask, oral mask, oro-nasal mask, full face mask, unsealed oro-nasal interface, nasal pillows mask, endotracheal tube, or other such respiratory interface.

Additionally, in accordance with certain features, aspects and advantages of one or more of the embodiments disclosed herein, a fixation structure for securing a patient interface on a face of a patient comprises a first edge; a second edge opposing the first edge, such that in use on the patient's face the second edge is farther away than the first edge from the patient's nose or mouth; and opposing third and fourth edges extending between the first and second edges. Portions of the third edge that are closer to the first and second edges project farther from a centerline or center portion of the fixation structure than do portions of the third edge that are farther from the first and second edges.

Portions of the third edge that are closer to the second edge can project farther from the centerline or center portion of the fixation structure than do portions of the third edge that are closer to the first edge. The third edge can comprise a concave curve. The fourth edge can comprise a concave curve shaped to match a shape of an under-eye region of the patient.

The fixation structure can comprise a first side extending between the first and second edges. The first side can face away from the patient's face and toward the patient interface in use. The first side can comprise a first fastener that is attachable to a complementary fastener of an attachment structure of the patient interface. The first fastener can be positioned closer to the first edge than to the second edge. A midpoint of the first fastener can be offset from a centerline or central portion midway between the first and second edges. A midpoint of the first fastener can be on a centerline or central portion midway between the third and fourth edges.

At least a portion of the first side not covered by the first fastener can comprise a gripping or bearing region. The first fastener can extend from the first edge to a position along a length of the first side between the first and second edges. A length of the first fastener between the first and second edges can be less than a length of the first side between the first and second edges. A ratio of the length of the first fastener to the length of the first side can be between 0.2 and 0.9.

The first fastener can extend substantially the entirety between the first and second edges and only partly between the third and fourth edges, and the first fastener can extend from the third edge to a position along a width of the first side between the third and fourth edges. The first fastener can extend from the first edge to a position along a length of the first side between the first and second edges, and the first fastener can extend from the third edge to a position along a width of the first side between the third and fourth edges.

The first fastener can comprise a hooked fastener that is removably attachable to a complementary looped fastener of the attachment structure of the patient interface. The hooked fastener can comprise a plurality of hooks that extend outwardly from the first side. At least some of the plurality of hooks can extend toward the first and/or second edges. At least some of the plurality of hooks can be generally parallel to the first and second edges and generally perpendicular to the third and fourth edges.

The first fastener can comprise a plurality of slits that facilitate flexibility of the first fastener. At least some of the plurality of slits can extend to at least one of the first, second, third, or fourth edges. At least some of the plurality of slits can be arranged in columns of slits that extend to the third and fourth edges in alternation. Each of the columns of slits can comprise slits with semicircular or crescent shapes that face the opposite direction to, and are at least partially offset toward the third or fourth edge from, semicircular or crescent shapes of slits in an adjacent column.

At least some of the plurality of slits can extend toward the third and fourth edges. At least some of the plurality of slits that are closer to the first and second edges can be shorter than at least some of the plurality of slits that are farther from the first and second edges. At least some of the plurality of slits can fail to extend across a centerline or central portion midway between the third and fourth edges.

At least one of the plurality of slits can have a serpentine shape. Two of the plurality of slits can have serpentine shapes, one of the serpentine slits can extend toward the first edge, and the other of the serpentine slits can extend toward the second edge.

At least some of the plurality of slits can have zig-zag shapes.

The fixation structure can comprise a second side extending between the first and second edges. The second side can face toward the patient's face and away from the patient interface in use. The second side can comprise a second fastener that is attachable to the patient's face. The second fastener can comprise an adhesive that is removably attachable to the face of the patient. The adhesive can be a hydrocolloid-based adhesive. The adhesive can be a silicone-based adhesive. The adhesive can comprise a material that in use substantially maintains a constant level of adherence to the patient's face for at least a predetermined period of time after application to the face. The adhesive can comprise a material that in use substantially conforms to the shape of the patient's face.

The fixation structure can comprise a material with low hygroscopicity. The fixation structure can comprise a material with a low Young's modulus. The fixation structure can have a substantially uniform thickness. The fixation structure can have a thickness that allows the fixation structure to flex in response to movements of the patient's face. The fixation structure can have a thickness that is between 0.05 mm and 5 mm.

Additionally, in accordance with certain features, aspects and advantages of one or more of the embodiments disclosed herein, a fixation structure for securing a patient interface on a face of a patient comprises a first edge; a second edge opposing the first edge, such that in use on the patient's face the second edge is farther away than the first edge from the patient's nose or mouth; a first side extending between the first and second edges, the first side facing away from the patient's face and toward the patient interface; and a second side opposing the first side. The first side comprises a first fastener, the first fastener attachable to a complementary fastener of an attachment structure of the patient interface. The second side comprises a second fastener attachable to the patient's face. The first edge is substantially straight. The first and second edges are substantially the same width.

The first and second edges can be substantially the same in shape and size. The second edge can be substantially rounded. The fixation structure can comprise a periphery with a substantially triangular shape. The fixation structure can comprise a periphery that is substantially curved. The fixation structure can comprise a substantially straight third edge extending between the first and second edges. The fixation structure can comprise opposing substantially straight third and fourth edges extending between the first and second edges.

Additionally, in accordance with certain features, aspects and advantages of one or more of the embodiments disclosed herein, a patient interface system comprises a fixation structure according to any one of the previously disclosed embodiments; and a patient interface comprising one or more attachment structures facing toward a patient's face in use. Each of the one or more attachment structures comprises a fastener that is attachable to a complementary fastener of the fixation structure.

The patient interface can comprise a first body adapted to rest on a first portion of the face. The patient interface can comprise a second body adapted to rest on a second portion of the face. The patient interface can comprise a bridge linking the first and second bodies. One or both of the first and second bodies each can comprise one of the attachment structures. One or both of the first and second bodies each can comprise a first edge and a second edge opposing the first edge. In use on the patient's face, the second edge can be farther away than the first edge from the patient's nose or mouth. The second edge can comprise a detachment structure to ease removal of the patient interface from the patient's face. The patient interface can comprise a nasal cannula, nasal mask, oral mask, oro-nasal mask, full face mask, unsealed oro-nasal mask, nasal pillows mask, or endotracheal tube

### BRIEF DESCRIPTION OF THE DRAWINGS

Specific embodiments and modifications thereof will become apparent to those skilled in the art from the detailed description herein having reference to the figures that follow.
Figure 1 shows a respiratory therapy system.
Figure 2 shows a front view of a patient interface.
Figure 3 shows a rear view of the patient interface shown in Figure 2.
Figure 4 shows a side perspective view of the patient interface shown in Figure 2.
Figure 5A shows a top view of a patient-facing region of a fixation structure.
Figure 5B shows a top view of an interface-facing region of a fixation structure.
Figure 5C shows a top view of an interface-facing region of a fixation structure.
Figure 6A shows a fixation structure fitted to a patient.
Figure 6B shows a fixation structure fitted to a patient.
Figure 7A shows a top view of an interface-facing region of a fixation structure.
Figure 7B shows a top view of an interface-facing region of a fixation structure.
Figure 7C shows a top view of an interface-facing region of a fixation structure.
Figure 7D shows a top view of an interface-facing region of a fixation structure.
Figure 7E shows a top view of an interface-facing region of a fixation structure.
Figure 7F shows a top view of an interface-facing region of a fixation structure.
Figure 7G shows a top view of an interface-facing region of a fixation structure.
Figure 8A shows a top view of an interface-facing region of a fixation structure.
Figure 8B shows a cross-section of an interface-facing region of a fixation structure along the lines A-A.
Figure 9A shows a top view of an interface-facing region of a fixation structure.
Figure 9B shows a cross-section of an interface-facing region of a fixation structure along the lines B-B.
Figure 10A shows a diagram of a fixation structure on a layer of skin.
Figure 10B shows a diagram of a fixation structure on a layer of skin.
Figures 11A-12H show top views of interface-facing regions of fixation structures.
Figure 13A shows a top view of an interface-facing region of a fixation structure.
Figure 13B shows a cross-section of the fixation structure of Figure 13A along line C-C.
Figure 14 shows a top view of an interface-facing region of another fixation structure.
Figure 15 shows a top view of an interface-facing region of another fixation structure.

### DETAILED DESCRIPTION

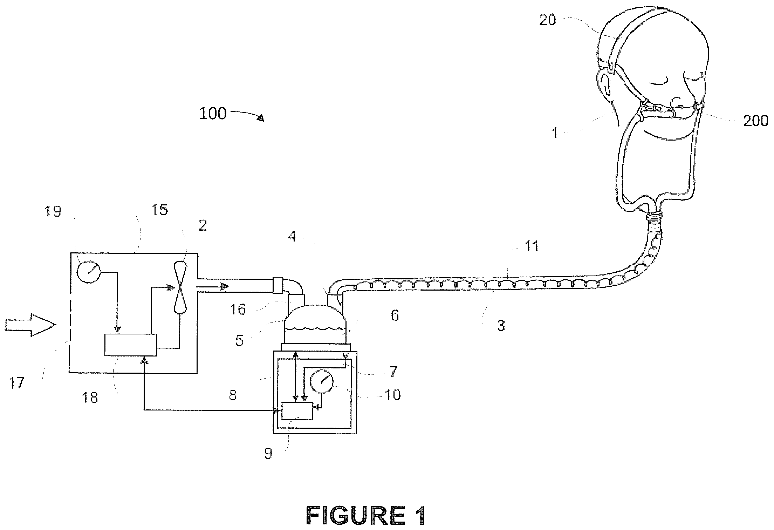

Figure 1 illustrates an example respiratory therapy system 100. A patient 1 is receiving heated and humidified gases through a patient interface 200, shown in this example as a nasal cannula assembly, connected to a humidified gases transportation pathway or inspiratory conduit 3 that in turn is connected to a humidifier 8 (including a humidification chamber 5) that is supplied with gases from a blower 15 or other appropriate gases supply. The gases can be supplied from a source that is external to and/or separate from the respiratory therapy system 100, or from a source that is internal to and/or integrated with the respiratory therapy system 100. Headgear 20 is provided to support and retain the patient interface 200 against the patient's face. The inspiratory conduit 3 is connected to an outlet 4 of the humidification chamber 5 which contains a volume of water 6. The humidification chamber 5 is preferably formed from a plastics material and may have a highly heat conductive base (for example an aluminum base) which is in direct contact with a heater plate 7 of the humidifier 8. The humidifier 8 is provided with a control mechanism or electronic controller 9 that may include a microprocessor based controller executing computer software commands stored in associated memory. Gases flowing through the inspiratory conduit 3 are passed to the patient's airway through the patient interface 200.

The controller 9 receives input from sources such as input means (e.g., a dial 10) through which a user (e.g., a nurse or other healthcare provider) may, for example, set a predetermined required value (preset value) of humidity or temperature of the gases supplied to the patient. In response to the user-set humidity or temperature value input via the dial 10 and other possible inputs such as internal sensors that sense gases flow or temperature, or parameters calculated by the controller 9, the controller 9 determines when (or to what level) to energize the heater plate 7 to heat the volume of water 6 within the humidification chamber 5. As the volume of water 6 within the humidification chamber 5 is heated, water vapor begins to fill the volume of the chamber above the water's surface and is passed out of the outlet 4 of the humidification chamber 5 with the flow of gases (for example air) provided from the gases supply means or blower 15 which enters the humidification chamber 5 through an inlet 16. It should be noted that it is possible to obtain the relationship between the humidity of the gases in humidification chamber 5 and the temperature of the heater plate 7. Accordingly, it is possible to utilize the temperature of the heater plate 7 in an algorithm or a look-up table to determine the humidity of the gases.

The blower 15 may be provided with a variable speed pump or fan 2 that draws air or other gases through the blower inlet 17. The speed of the variable speed pump or fan 2 may be controlled by a further control means or electronic controller 18 (or alternatively the function of the controller 18 could be carried out by the controller 9) in response to inputs from the controller 9 and a user-set predetermined required value (preset value) of pressure and/or fan speed via one or more input devices, such as a dial 19.

A heating element 11 may be provided within the conduit 3 to help prevent condensation of the humidified gases within the conduit 3. Such condensation is due to the temperature of the walls of the conduit 3 being close to the ambient temperature, (being the temperature of the surrounding atmosphere) which is usually lower than the temperature of the humidified gases within the conduit 3. The heating element 11 effectively replaces the energy lost from the gases through conduction and convection during transit through the conduit 3. Thus the heating element 11 ensures the gases delivered are at an optimal temperature and humidity.

In the illustrated configurations, the patient interface 200 includes a nasal cannula. In some alternative configurations, the patient interface 200 may include a sealing or non-sealing interface. For example, the patient interface 200 may include a nasal mask, an oral mask, an oro-nasal mask, a full face mask, a nasal cannula, an unsealed oro-nasal interface, a nasal pillows mask, an endotracheal tube, a combination of the above, or some other gases conveying system or apparatus. Certain features, aspects and advantages of the illustrated nasal cannula may be envisaged in other such patient interfaces.

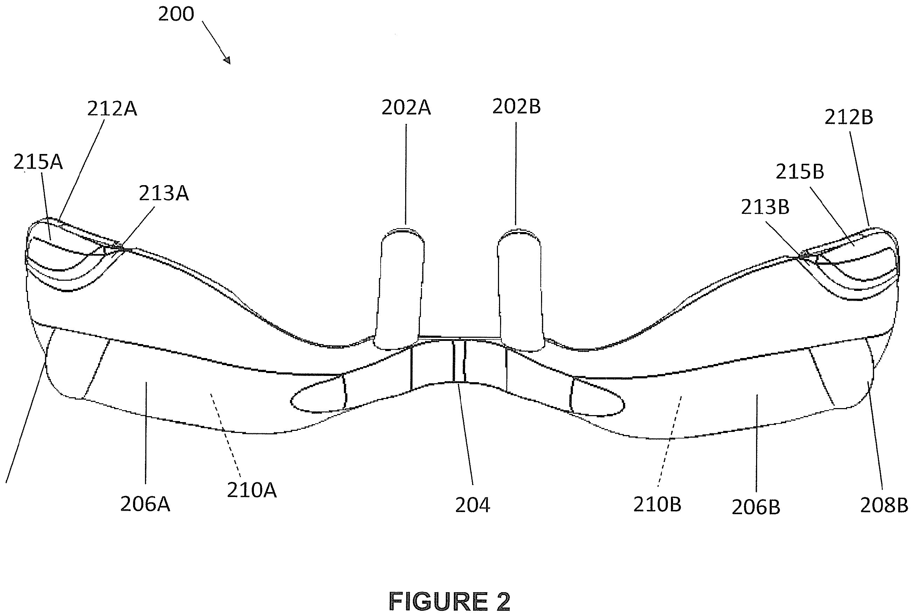

Referring to Figures 2-4, the patient interface 200 is shown. The patient interface 200 can be generally shaped or configured such that it substantially matches the contours of the face. The patient interface 200 includes first and second nasal delivery elements 202A, 202B adapted to rest in the patient's nares. The illustrated nasal delivery elements 202A, 202B are substantially tubular and direct gases passing through the patient interface 200 to the patient. The nasal delivery elements 202A, 202B can be shaped and angled such that they generally extend inwardly toward the patient's septum in use. The nasal delivery elements 202A, 202B end in tips 202A', 202B'. In use, the tips 202A', 202B' point toward the back of the patient's head. It should be understood that in some configurations, the nasal delivery elements 202A, 202B could have different shapes. For example, although the average cross-section of the nasal delivery elements 202A, 202B in the illustrated configurations is substantially circular, in some configurations the cross-section of the nasal delivery elements 202A, 202B could be substantially ellipsoidal, substantially square, or substantially rectangular. In some configurations, the cross-section of the nasal delivery elements 202A, 202B could vary along the length of the nasal delivery elements 202A, 202B. In some configurations, the first and second nasal delivery elements 202A, 202B may have different characteristics. For example, the first nasal delivery element 202A may be smaller or shorter than the second nasal delivery element 202B. In some configurations, only one nasal delivery element may be used. In some configurations, more than two (for example, three or four) nasal delivery elements may be used.

The first and second nasal delivery elements 202A, 202B extend from first and second bodies 206A, 206B of the patient interface 200. The first and second bodies 206A, 206B include internal gases delivery lumens 210A, 210B, that receive gases from gases inlets 208A, 208B of the first and second bodies 206A, 206B, and channel the gases to the first and second nasal delivery elements 202A, 202B. The gases inlets 208A, 208B couple with a pair of gases delivery conduits 218A, 218B (see Figure 1). In the illustrated configuration, the gases delivery conduits 218A, 218B are integrally formed or inseparably connected to the gases inlets 208A, 208B. The gases delivery conduits 218A, 218B in turn are integrally formed or inseparably connected to a gases conduit connector 222. The gases conduit connector 222 releasably couples with a complementary connector 118 in pneumatic communication with the gases conduit 110 (described elsewhere in this disclosure with reference to Figure 1 as inspiratory conduit 3). Other configurations are contemplated. For example, in some configurations, the first and second nasal delivery elements 202A, 202B may receive gases from a single internal gases delivery lumen positioned in one of the first or second bodies 206A, 206B. The single internal gases delivery lumen in turn may receive gases from a single gases delivery conduit.

In some configurations, the one or more gases inlets 208A, 208B may couple directly with the gases conduit 110 (or indirectly via the complementary connector 118 and/or the gases conduit connector 222). In some such configurations, the first and/or second bodies 206A, 206B may be integrally formed or may be in the form of a single continuous assembly together with the gases conduit 110. In some configurations one or more of the gases delivery conduits 218A, 218B are removably coupled to one or more of the gases inlets 208A, 208B. In some configurations one or more of the gases delivery conduits 218A, 218B are removably coupled to the gases conduit connector 222.

The first and second bodies 206A, 206B of the patient interface 200 are adapted to rest on the patient's face. In the illustrated configuration, the first and second bodies 206A, 206B are adapted to rest on opposing cheeks of the patient's face. To fix the first and second bodies 206A, 206B in place on the patient's face, patient-facing regions of the first and second bodies 206A, 206B are provided with attachment structures 216A, 216B. The attachment structures 216A, 216B are adapted to maintain the patient interface 200 in a desired alignment with the face, such that the nasal delivery elements 202A, 202B may, in a non-limiting example, be comfortably and non-sealingly positioned in the nares.

The attachment structures 216A, 216B couple with or attach to fixation structures that are adhered to the face in use. In some configurations, the attachment structures 216A, 216B and the fixation structures can be structured such that they are complementary to one another. For example, the attachment structures 216A, 216B may include 'loop' portions (constructed from, for example, textiles or plastics) that are adhered or otherwise secured to patient-facing regions of the first and second bodies 206A, 206B, and the fixation structures may be adhered or otherwise secured to the face and may include 'hook' pads. The attachment structures 216A, 216B and the fixation structures couple or attach to each other via, for example, a 'hook-and-loop' style connection. Other configurations are contemplated. In some configurations, the attachment structures 216A, 216B may interface directly with the face, for example, through the use of adhesive pads or other structures. In some configurations, a single attachment structure may be used, or more than two, for example, three or four, attachment structures may be used.

In some configurations, the attachment structures 216A, 216B may include features other than 'loop' portions. For example, the attachment structures 216A, 216B may include 'hook' portions that couple with or attach to complementary 'loop' portions on the fixation structures. As another example, the attachment structures 216A, 216B may include snap-fit features or other mechanical interlock features that couple with or attach to features on the fixation structures. In some configurations, the patient interface 200 may additionally include headgear adapted to retain the patient interface 200 in a desired orientation or alignment on the face. The headgear may include, for example, one or more straps configured to extend around the patient's head, buckles to adjust the tightness of the straps by modifying the effective length of the straps, caps, coifs, hats, helmets, and/or one or more other features.

A bridge 204 connects the first and second bodies 206A, 206B. The bridge 204 is an extension of the first and/or second bodies 206A, 206B. In some embodiments, the bridge 204 includes no internal gases lumen. The bridge 204 serves to help keep the nasal delivery elements 202A, 202B in a desired orientation with respect to one another. Other configurations are contemplated. For example, in some configurations, the bridge 204 could be constructed from a different material to the first and second bodies 206A, 206B. Alternatively, the bridge 204 could be constructed from the same material as the first and second bodies 206A, 206B. In some configurations, the bridge 204 may be open to provide transmission of gases between the first and second bodies 206A, 206B, for example, via an internal gases lumen that fluidly couples the nasal delivery elements 202A, 202B. In some such configurations, only one of the bodies 206A, 206B includes an internal gases lumen and/or gases inlet. In some configurations, the bridge 204 extends between a first connection point on the first body 206A and a second connection point on the second body 206B.

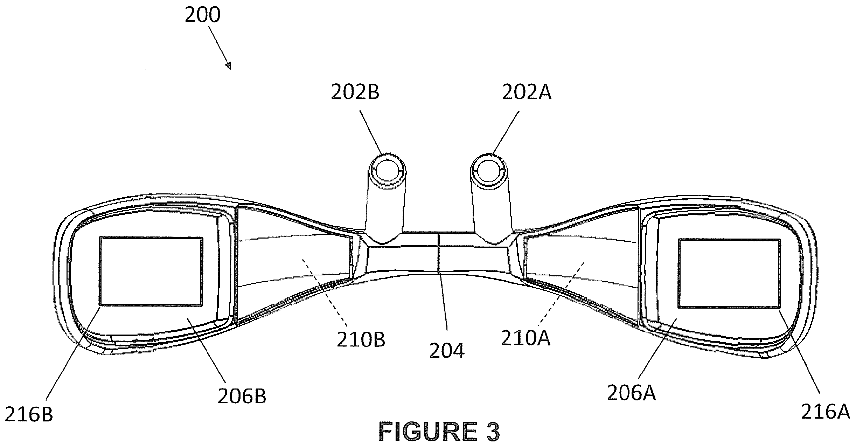

As seen in Figure 3, the first and second bodies 206A, 206B may decrease in width as they extend toward the nasal delivery elements 202A, 202B and the bridge 204. The attachment structures 216A, 216B are positioned on relatively wide portions of the first and second bodies 206A, 206B at or near the outer edges of the first and second bodies 206A, 206B to improve stability of the patient interface 200 on the face when the attachment structures 216A, 216B are used together with the fixation structures. For example, the attachment structures 216A, 216B can be positioned at or near distal portions of the first and second bodies 206A, 206B relative to the nasal delivery elements 202A, 202B. The attachment structures 216A, 216B may substantially cover the patient-facing regions of the first and second bodies 206A, 206B. The attachment structures 216A, 216B may at least partially cover the patient-facing regions of the first and second bodies 206A, 206B. The attachment structures 216A, 216B can be positioned within at least partially recessed regions of the first and second bodies 206A, 206B.

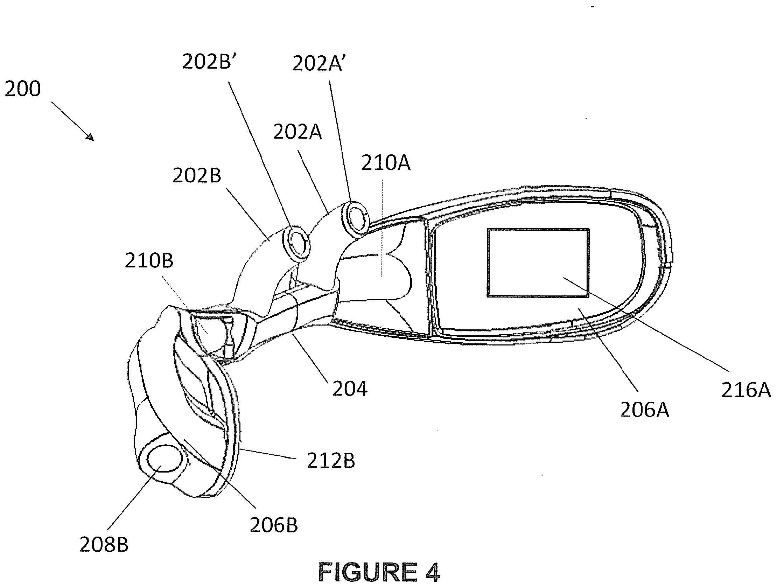

With further reference to Figure 2 and 4, outer edges of the first and second bodies 206A, 206B can include detachment structures 212A, 212B. The detachment structures 212A, 212B are positioned on a non-patient-facing region of the bodies 206A, 206B. The detachment structures 212A, 212B are spaced apart from the gases inlets 208A, 208B. In the illustrated configuration, the detachment structures 212A, 212B are tabs that include inner regions of reduced thickness relative to adjacent portions of the bodies 206A, 206B. Outside of the inner regions of the tabs and toward the outer edge of the bodies 206A, 206B, the tabs additionally include outer regions of normal thickness or increased thickness relative to the same adjacent portions of the bodies 206A, 206B. The outer regions may be grasped by the patient or another person (for example, a healthcare professional such as but not limited to a nurse or physician) and pulled. When pulled, the outer regions may rotate around the inner regions and sufficient forces may be exerted on the patient interface 200 such that the attachment structures 216A, 216B are detached from the fixation structures present on the face. The detachment structures 212A, 212B thus may be used to more easily detach the patient interface 200 from the face. Other configurations are contemplated. For example, in some configurations, one of the detachment structures 212A, 212B may be positioned on one of the first or second bodies 206A, 206B of the patient interface 200. In some configurations, the patient interface 200 may include more than two, for example, three or four, detachment structures. In some configurations, the detachment structures 212A, 212B may be positioned on other portions of the bodies 206A, 206B, or on other portions of the patient interface 200. In some configurations, the detachment structures 212A, 212B may include structures other than tabs. For example, the detachment structures 212A, 212B may include flat extensions of the first and/or second bodies 206A, 206B that can be pulled or otherwise manipulated to separate the patient interface 200 from the face.

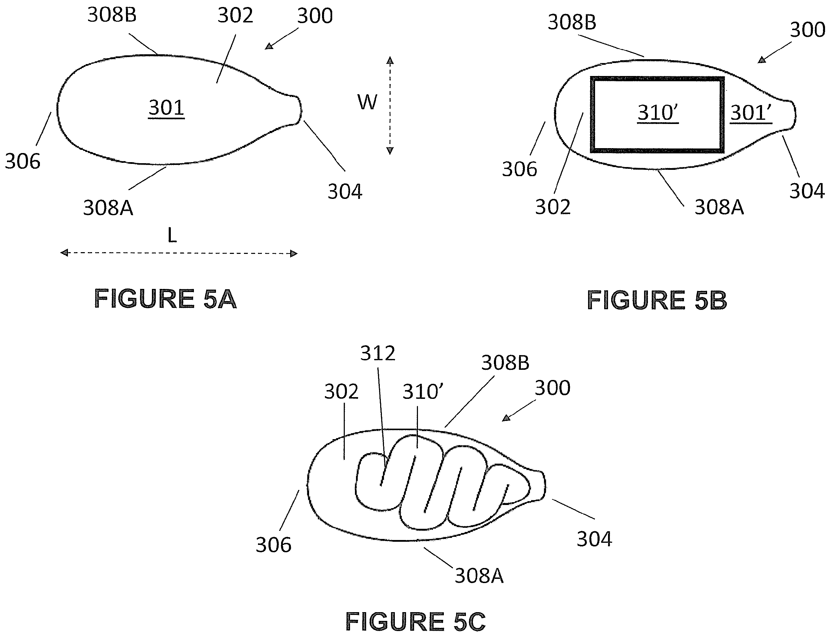

Figure 5A shows a patient-facing region 301 of a fixation structure 300, and Figure 5B shows an interface-facing region 301' of the fixation structure 300. The patient-facing region 301 removably couples with or adheres to the patient's face. The interface-facing region 301' of the fixation structure 300 attaches to the patient interface 200 to removably couple the patient interface 200 to the patient's face. The fixation structure 300 removably joins with the attachment structures 216A, 216B as described elsewhere in this disclosure with reference to Figures 2 - 4.

In the illustrated configuration, the fixation structure 300 includes a body 302. The body 302 is of substantially uniform thickness. In other configurations, the body 302 may be of variable thickness along the length L and/or width W of the body 302. The body 302 includes a first edge 304 adapted to face toward the patient's nose or mouth in use. In the illustrated configuration, the first edge 304 is substantially rounded and tapers in width to form an extension or knob of the body 302. The body 302 additionally includes a second edge 306, which is a lateral edge with respect to the patient's face, and therefore adapted to face away from the patient's nose or mouth in use. In the illustrated configuration, the second edge 306 is substantially rounded and slightly tapers in width. Opposed curved third and fourth edges 308A, 308B extend between the first and second edges 304, 306 of the body 302.

The interface-facing region 301' of the fixation structure 300 includes an interface-facing fixation element 310'. The interface-facing fixation element 310' engages with the attachment structures 216A, 216B to secure the patient interface 200 to the patient's face in use. In the illustrated configuration, the interface-facing fixation element 310' is a hooked pad that removably joins with a looped pad of one of the attachment structures 216A, 216B (e.g. for a hook-and-loop style connection). The hooked pad may be joined to the fixation structure 300 by a number of means, including but not limited to the use of adhesives, ultrasonic welding, high frequency welding, stitching, or chemical bonding. In other configurations, the interface-facing fixation element 310' may include other structures or elements adapted to couple with or attach to the attachment structures 216A, 216B, including but not limited to catches, which can couple with complementary latches of the attachment structures 216A, 216B or latches to couple with or attach to complementary catches of the attachment structures 216A, 216B, complementary adhesives, pins, clasps, or other mechanical fasteners.

The patient-facing region 301 is constructed from a material that can be removably joined to the patient's face. In the illustrated configuration, the material is an adhesive material, for example a hydrocolloid-based adhesive material, a zinc oxide-based adhesive material, or a hydrogel-based material.

Figure 5C shows an exemplary embodiment of the fixation structure 300 and the interface-facing fixation element 310'. In the illustrated configuration, the interface-facing fixation element 310' is a hooked pad with a serpentine shape. The serpentine shape defines gaps 312 between adjacent masses of the interface-facing fixation element 310'. The gaps 312 improve the flexibility of the interface-facing fixation element 310', particularly when the fixation structure 300 is placed under torsion around an axis substantially parallel to the first and/or second edges 304, 306. The gaps 312 can include cuts, slits, fenestrations, or thin regions formed in the interface-facing fixation element 310'. The interface-facing fixation element 310' can be configured to exhibit increased flexibility under torsion around the axis substantially parallel to the first and/or second edges 304, 306 by altering the material configuration. For example, in some configurations the interface-facing fixation element 310' could be constructed from a mechanically anisotropic material that exhibits increased flexibility when under torsion around the axis substantially parallel to the first and/or second edges 304, 306. In some configurations, the interface-facing fixation element 310' could be constructed from multiple materials of varying flexibility. The multiple materials could be stratified along the length of the interface-facing fixation element 310'.

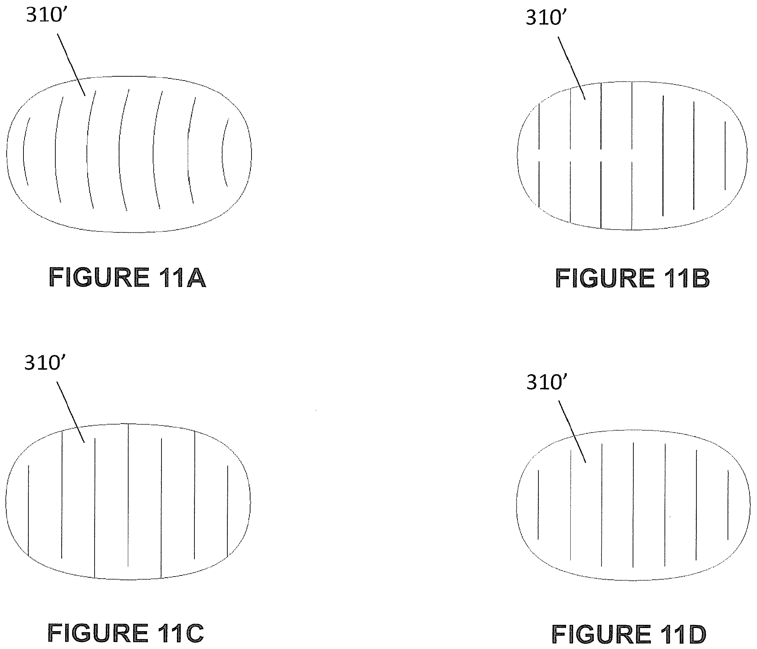

Figures 11A-11D illustrate the use of various patterns, for example, of slits, for the interface-facing fixation element 310' . The interface-facing fixation element 310' has a substantially elliptical shape, although in other configurations the interface-facing fixation element 310' can have other shapes, including, but not limited to, rectangular, square, trapezoidal, triangular, or pentagonal shapes. As described with reference to Figure 5C, the interface-facing fixation element 310' can include slits or other features that improve the flexibility of the interface-facing fixation element 310'. Various patterns of slits can be used to impart flexibility to the interface-facing fixation element 310'. In some embodiments, the slits extend to at least one edge of the interface-facing fixation element 310'. In some embodiments, the slits do not extend to an edge of the interface-facing fixation element 310'.

Figure 11A shows an embodiment of the interface-facing fixation element 310' including a pattern of successive slits that arcuately extend toward a lengthwise edge of the interface-facing fixation element 310'. The slits increase in length toward a center of the interface-facing fixation element 310'.

Figure 11B shows an example of the interface-facing fixation element 310' including a pattern of pairs of slits in a first region of the interface-facing fixation element 310' and singular slits in a second region of the interface-facing fixation element 310'. The pairs of slits in the first region of the interface-facing fixation element 310' extend substantially inwardly from the third and fourth edges 308A, 308B of the interface-facing fixation element 310' and increase in length toward a center of the interface-facing fixation element 310'. The singular slits in the second region of the interface-facing fixation element 310' increase in length toward the center of the interface-facing fixation element 310'.

Figure 11C shows an example of the interface-facing fixation element 310' including a pattern of slits that alternately extend from opposing widthwise edges of the interface-facing fixation element 310'. The slits increase in length toward a center of the interface-facing fixation element 310'.

Figure 11D shows an example of the interface-facing fixation element 310' including a pattern of successive straight slits that increase in length toward a center of the interface-facing fixation element 310'.

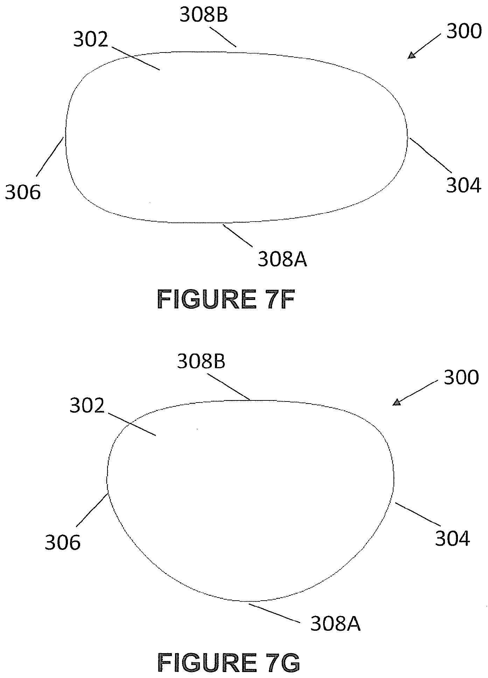

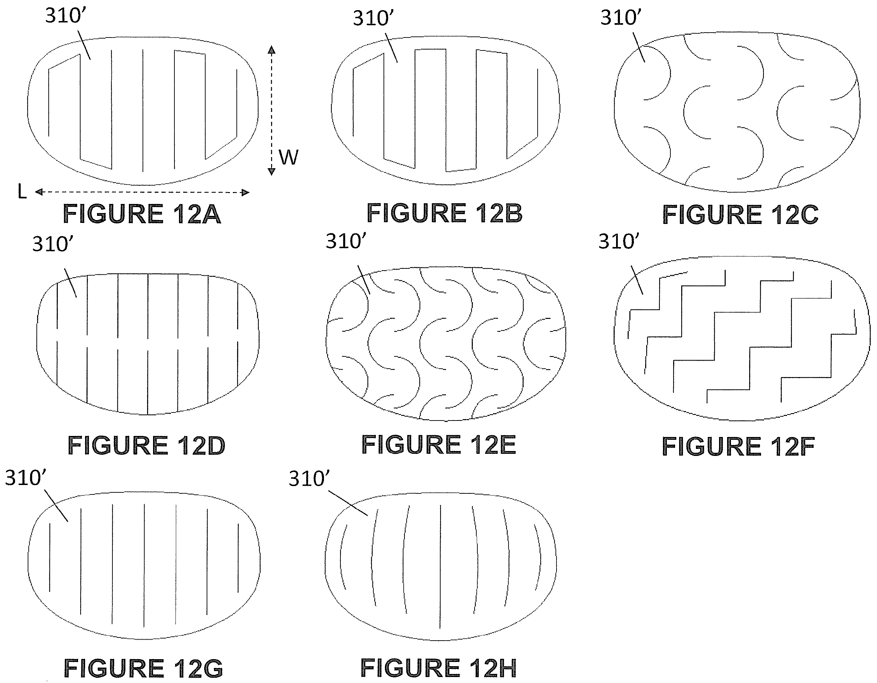

Figures 12A-12K illustrate the use of various patterns of slits on embodiments of the interface-facing fixation element 310' having a substantially triangular shape, or a shape similar to the Reuleaux triangle-like shape with rounded edges or 'guitar pick'-like shape shown in Figure 7G. However, it should be understood that such patterns of slits could be similarly applied to fixation elements having other shapes. In some embodiments, the slits extend to at least one edge of the interface-facing fixation element 310'. In some embodiments, the slits do not extend to an edge of the interface-facing fixation element 310'.

Figure 12A shows an example of the interface-facing fixation element 310' including a pattern of a pair of slits extending from opposing ends of the interface-facing fixation element 310'. As shown, the slits can extend along a portion of the width W and length L of the interface-facing fixation element 310'. In some embodiments, each of the pair of slits has a serpentine shape. The slits can have straightened edges defining each turn or change in direction of the serpentine slit. As illustrated, one or each of the serpentine slits can include substantially parallel widthwise sections linked by lengthwise sections. The lengthwise sections can extend along the edges of the interface-facing fixation element 310', and can extend in a radially outward direction, and/or can extend toward a center of the interface-facing fixation element 310'. In some configurations, the lengthwise sections are at an angle substantially offset from a lengthwise direction (e.g., a longitudinal axis of the interface-facing fixation element 310'). As shown, some configurations have a single, non-serpentine slit that can be positioned between the pair of slits. The single slit can extend at least partially between the third and fourth edges 308A, 308B.

Figure 12B shows an example of the interface-facing fixation element 310' including a pattern of a single slit having a serpentine shape with straightened edges defining each turn or change in direction of the serpentine slit.

Figure 12C shows an example of the interface-facing fixation element 310' including a pattern of alternating columns of semicircular slits. Each column includes semicircular slits with shapes that face the opposite direction to, and are at least partially offset from, the columns on either side.

Figure 12D shows an example of the interface-facing fixation element 310' including a pattern of slits that extend from the third and fourth edges 308A, 308B of the interface-facing fixation element 310' inwardly toward a center of the interface-facing fixation element 310'. The illustrated slits may not extend across the entirety of the interface-facing fixation element 310'.

Figure 12E shows an example of the interface-facing fixation element 310' including a pattern similar to that shown in Figure 12C. However, in the illustrated interface-facing fixation element 310', the columns of semicircular slits are aligned such that they are closer together than the columns shown in Figure 12C. In an embodiment, the semicircular slits can overlap.

Figure 12F shows an example of the interface-facing fixation element 310' including a pattern of slits that include 'stair step' or zig-zag shapes.

Figure 12G shows an example of the interface-facing fixation element 310' including a pattern of successive straight slits that increase in length toward a center of the interface-facing fixation element 310'. The slits are substantially parallel with the second edge 306 of the interface-facing fixation element 310'.

Figure 12H shows an example of the interface-facing fixation element 310' including a pattern similar to that shown in Figure 12G. However, in the illustrated interface-facing fixation element 310', the centermost slit is straight and substantially parallel to the second edge 306 of the interface-facing fixation element 310', and the other slits curve inwardly toward the centermost slit.

Although the illustrated embodiments of the fixation structure 300 are useful in aiding in the retention of the patient interface 200 on the patient's face, in some cases forces applied to the fixation structure 300 can cause the fixation structure 300 to be unintentionally lifted from the patient's face, which can be an inconvenience to the patient and/or a medical professional tending to the patient. This may occur because a portion of the forces applied to remove the patient interface 200 from the fixation structure 300 may be transferred to the fixation structure 300. Force may be applied, for example, in a direction normal to the patient's cheek. To reduce the likelihood of unintentional removal of the fixation structure 300 from the patient's cheek, such as upon removal of the patient interface 200, the adhesion of the fixation structure 300 to the patient's cheek may be greater than the adhesion of the fixation structure 300 to the patient interface 200. To remove the fixation structure 300 from the patient's face, a removal force may be applied that overcomes the adhesion between the patient's face and the fixation structure 300. Alternatively, removal mechanisms, such as application of a remover such as water, could reduce the adhesion between the patient's face and the fixation structure 300. Other removers are also contemplated.

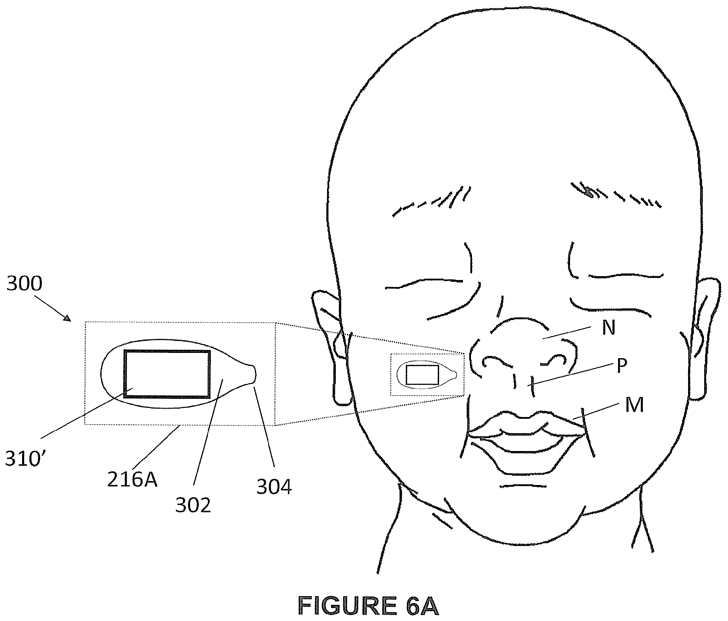

Figure 6A shows the fixation structure 300 with the patient-facing region 301 secured to the patient's face. In other words, the patient-facing region 301 is facing into the page in Figure 6A. As shown, the first edge 304 of the fixation structure 300 faces toward the nose N, philtrum P, and/or mouth M of the patient. The interface-facing fixation element 310' is adapted to couple with or attach to the attachment structure 216A of the first body 206A of the patient interface 200. In some configurations, when properly aligned with the interface-facing fixation element 310', the bodies 206A, 206B and/or the bridge 204 of the patient interface 200 may rest above the mouth M and over the philtrum P of the patient.

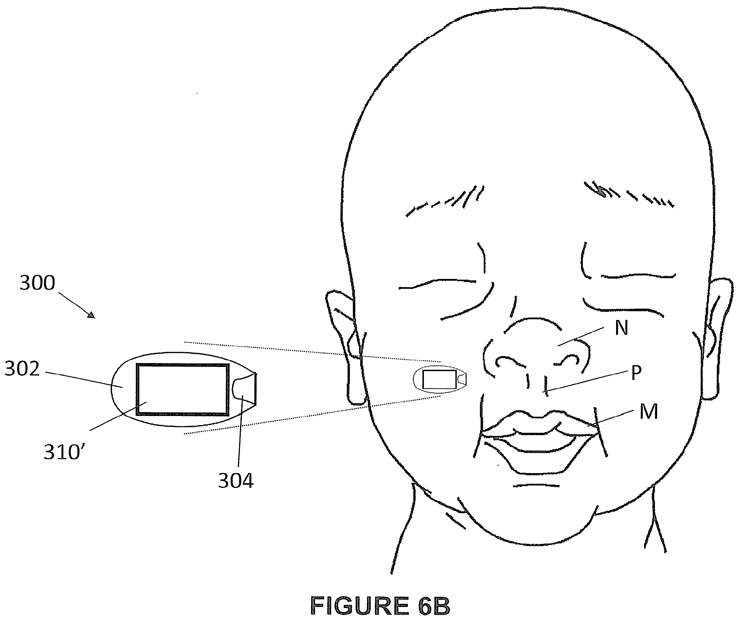

In some cases it may be desirable to detach the patient interface 200, which is positioned on the patient's face by attachment to the fixation structure 300, while keeping the fixation structure 300 on the patient's face. For example, this may be desirable when cleaning and/or replacing the patient interface 200, or securing tubes, including but not limited to, nasogastric or nasojejunal tubes onto the face. However, insufficient securement strength of the fixation structure 300 to the patient's face has been found to cause a part of the fixation structure 300 to be prone to being lifted away from the face. For example, the fixation structure 300 may be lifted away from the patient's face in response to a lifting force (e.g., a force that is substantially normal to the fixation structure 300) being exerted on the interface-facing fixation element 310'. In some configurations, lifting forces may be applied to the fixation structure 300 while the patient interface 200 is being removed from the fixation structure 300. The securement strength between the fixation structure 300 and the patient's face may depend on, for example, the adhesion of the adhesive or sticky material of a patient-facing fixation element 310 of the patient-facing region 301. In some cases, the entirety of the patient-facing fixation element 310, and thus the fixation structure 300, can be detached from the face. In some embodiments, when the lifting force is exerted on the interface-facing fixation element 310', the lifting force can be transferred to the patient-facing fixation element 310 (which is facing into the page in Figure 6B). As illustrated in Figure 6B, this transfer of force can cause the first edge 304, or other portions of the body 302, of the fixation structure 300 to be lifted from the face.

It has been found that the shape of one or more portions of the fixation structure 300 can be a factor in the ability of the fixation structure 300 to resist being lifted from the face, for example, upon removal of the patient interface 200. In particular, in the embodiments of the fixation structure 300 illustrated in Figures 5A - 6B, the tapered first edge 304 serves to concentrate pressure created by the lifting force exerted on the fixation structure 300, causing it to be vulnerable to being lifted from the face. In an embodiment, the shape of the fixation structure 300 can be chosen to determine the pressure distribution profile as the fixation structure 300 is removed from the face.

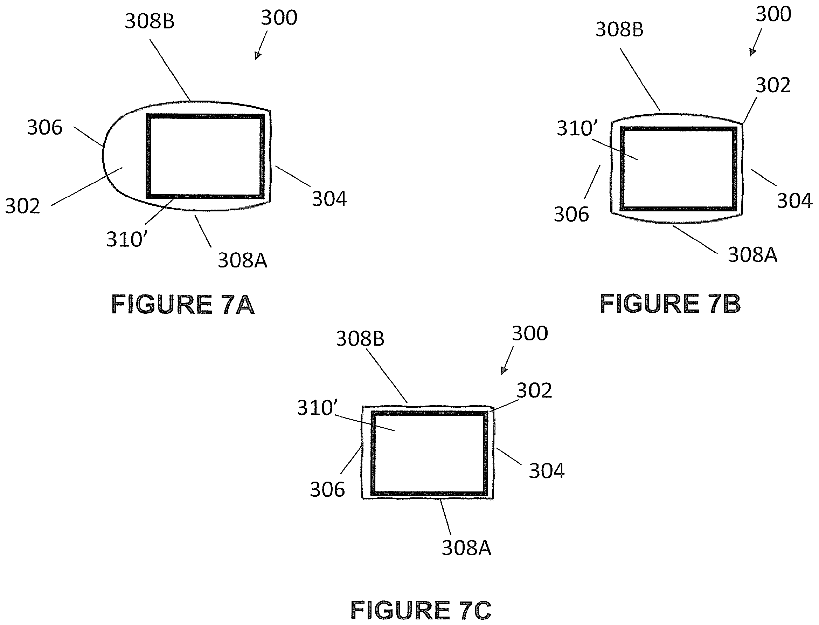

Figures 7A - 7E show non-limiting examples of embodiments of the fixation structure 300 and the interface-facing fixation element 310'. The body 302 of the various illustrated embodiments of the fixation structure 300 includes a substantially flat or straight embodiment of the first edge 304, rather than a first edge that tapers or narrows in width, as was described in connection with some of the embodiments above. The flat or straight first edge 304 allows pressure created by the lifting force to be distributed along the length of the first edge 304. In an embodiment, the corners of the fixation structure 300 are substantially rounded. In an embodiment, the corners of the interface-facing fixation element 310' are substantially rounded.

Figure 7A shows an example embodiment of the fixation structure 300 where the second edge 306 is rounded. The third and fourth edges 308A, 308B are at least partially rounded. The interface-facing fixation element 310' extends substantially along the entirety of the interface-facing region 301' of the body 302 in a direction defined between the third and fourth edges 308A, 308B. The interface-facing fixation element 310' extends partially along the interface-facing region 301' of the body 302 in a direction defined between the first edge 304 and the second edge 306. As a result, a portion of the second edge 306 may not be immediately adjacent to the interface-facing fixation element 310'.

Figure 7B shows an example embodiment of the fixation structure 300 where the second edge 306 is substantially flat or straight. The third and fourth edges 308A, 308B are substantially rounded. The interface-facing fixation element 310' covers substantially the entirety of the interface-facing region 301' of the body 302. Alternatively, the interface-facing fixation element 310' can cover at least a portion of the interface-facing region 301'. The interface-facing region 301' may provide a border or edge around the interface-facing fixation element 310'. A user (e.g., a nurse or other healthcare provider) may grasp an edge of the fixation structure 300 to remove the fixation structure 300 from the patient's face. The size of the interface-facing fixation element 310' relative to the size of the fixation structure 300 can be configured such that the forces applied to the fixation structure 300 during removal of the patient interface 200 do not cause the fixation structure 300 to be removed from the patient's face.

Figure 7C shows an example embodiment of the fixation structure 300 in which the first, second, third and fourth edges 304, 306, 308A, 308B are all substantially flat or straight. In an example, the fixation structure 300 forms a substantially square or a substantially rectangular shape. In an alternative example, the corners of the fixation structure 300 are substantially rounded. The interface-facing fixation element 310' covers substantially the entirety of the interface-facing region 301' of the body 302. Alternatively, the interface-facing fixation element 310' covers at least a portion of the interface-facing region 301'. The interface-facing region 301' may provide a border or edge around the interface-facing fixation element 310'. A user (e.g., a nurse or other healthcare provider) may grasp an edge of the fixation structure 300 to remove the fixation structure 300 from the patient's face. The size of the interface-facing fixation element 310' relative to the size of the fixation structure 300 can be configured such that the forces applied to the fixation structure 300 during removal of the patient interface 200 do not cause the fixation structure 300 to be removed from the patient's face.

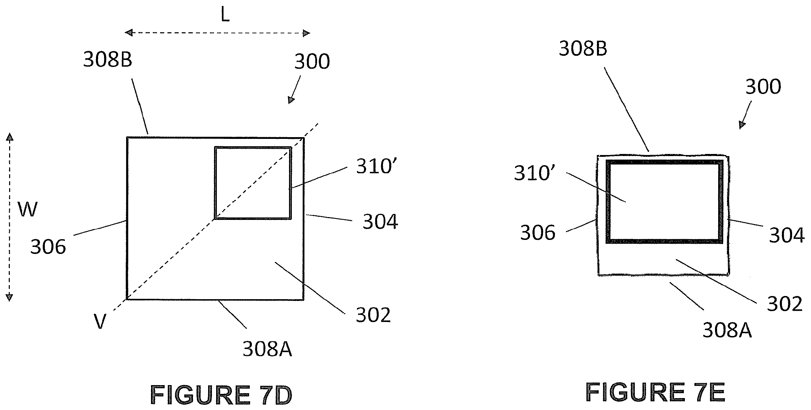

Figure 7D shows an example embodiment of the fixation structure 300 in which the first, second, third and fourth edges 304, 306, 308A, 308B are all substantially flat or straight. The interface-facing fixation element 310' covers only a portion of the interface-facing region 301' of the body 302. In particular, the interface-facing fixation element 310' extends from the first edge 304 to a position along the length L of the body 302 between the first and second edges 304, 306, and extends from the fourth edge 308B to a position along the width W of the body 302 between the third and fourth edges 308A, 308B. As illustrated, in some configurations, the body 302 and/or the interface-facing fixation element 310' have a generally square peripheral shape. In some configurations, the interface-facing fixation element 310' may be substantially symmetrical about a plane extending along a diagonal line V. As shown, in some configurations, the diagonal line V extends between the vertex defined by the first and fourth edges 304, 308B and the vertex defined by the second and third edges 306, 308A. The fixation structure 300 may then be rotatable by 90 degrees to be placed on either cheek of the face. Embodiments of the fixation structure 300 with substantially symmetrical construction (e.g., peripheral shape) can enhance convenience and/or can reduce the number of different parts for a healthcare facility to purchase, stock, and maintain.

Figure 7E shows an example embodiment of the fixation structure 300 in which the first, second, third, and fourth edges 304, 306, 308A, 308B are all substantially flat or straight. The interface-facing fixation element 310' extends along substantially the entirety of the length of the interface-facing region 301' of the body 302 between the first and second edges 304, 306. The interface-facing fixation element 310' only extends partially along the width of the interface-facing region 301' of the body 302 from the fourth edge 308B to a position between the third and fourth edges 308A, 308B.

Figure 7F and Figure 7G show non-limiting configurations of the fixation structure 300. Figure 7F shows an embodiment of the fixation structure 300 having a substantially elliptical shape where all the edges are substantially rounded. The first edge 304 tapers slightly along its length but the taper does not end in an extension or knob like the fixation structures shown in Figures 5A-6B. The curvature of the first edge 304 is such that an aesthetically pleasing shape is imparted while avoiding an undesirably concentrated pressure upon the first edge 304 when the lifting force is applied to the fixation structure 300.

Figure 7G shows an embodiment of the fixation structure 300 having a substantially triangular shape, or a shape similar to the Reuleaux triangle-like shape with rounded edges or a 'guitar pick'-like shape. The shape is substantially symmetrical and can be located on either side of the nose and have the same mechanical characteristics without requiring rotation or re-orientation. Similarly, the first and second edges 304, 306 (in this case, either in use being adapted to face toward the patient's nose and/or mouth) are rounded and taper slightly but still avoid undesirably concentrated pressures upon the first and second edges 304, 306 when the lifting force is applied to the fixation structure 300.

Other useful shapes for the fixation structure 300 are contemplated. In some patients, facial distortion (due to, for example, crying or expression of emotions) or improper handling of the fixation structure 300 can cause the fixation structure 300 to move over an eye or eyelid, potentially causing momentary duress or irritation for the patient. In some cases, it is desired to form the fixation structure 300 such that it is less likely to be pushed into the patient's eye when shear forces, for example, forces that urge the fixation structure 300 toward the eye, are exerted on the body 302 of the fixation structure 300.

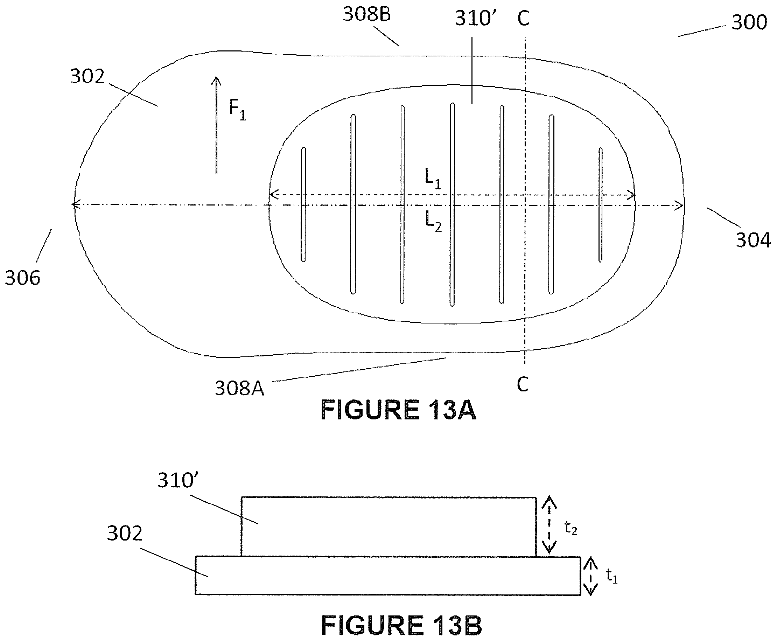

Figure 13A shows an interface-facing view of a non-limiting configuration for the fixation structure 300. The illustrated embodiment of the fixation structure 300 includes an embodiment of the first edge 304 that faces toward the patient's nose or mouth in use and an embodiment of the second edge 306 that faces away from the patient's nose or mouth in use. The opposed third and fourth edges 308A, 308B extend between the first and second edges 304, 306. As shown, the third and fourth edges 308A, 308B can include concave curves (e.g., with respect to a longitudinal axis of the fixation structure 300). As illustrated, in some embodiments, the third and fourth edges 308A, 308B include curves that curve or face in opposite directions. The third and fourth edges 308A, 308B project farther from a centerline or center portion of the body 302 at parts of the third and fourth edges 308A, 308B that are proximal to the first and second edges 304, 306 than at parts of the third and fourth edges 308A, 308B that are distal from the first and second edges 304, 306 (e.g. central portions of the third and fourth edges 308A, 308B). Portions of the third and fourth edges 308A, 308B proximal to the second edge 306 project farther from the centerline or center portion of the body 302 than portions of the third and fourth edges 308A, 308B proximal to the first edge 304. The concave curve of the fourth edge 308B substantially corresponds with a contour of an eye or eyelid, such as the lower eyelid. When an upwards force is exerted on the body 302 of the fixation structure 300, such as that shown along force vector F₁, the concave curvature of the fourth edge 308B increases the tendency of the body 302 of the fixation structure 300 to cup or move around the eye region. This can allow the body 302, for example, to cup the contour of the lower eyelid and/or can decrease the tendency of the body 302 to move into or onto the eye or eyelid. As the concave curve is present on both the third and fourth edges 308A, 308B, the fixation structure 300 can be rotated by approximately 180 degrees to be used on either side of the face while retaining the same properties.

As shown, the interface-facing fixation element 310' is on the interface-facing region 301' of the fixation structure 300 and thus, faces away from the patient's face. The interface-facing fixation element 310' is offset from a central portion of the body 302. In the illustrated configuration, the interface-facing fixation element 310' is positioned such that it is closer to the first edge 304 than to the second edge 306. In addition, the length L₁ of the interface-facing fixation element 310' is less than or equal to the length L₂ of the body 302 of the fixation structure 300, where L₂ is defined as the length between the first and second edges 304, 306. In some configurations, the ratio of the lengths L₁:L₂ might be in the range of 0.2 - 0.9, or in the range of 0.3 - 0.8, or in the range of 0.4 - 0.7, or in the range of 0.5 - 0.6. If the interface-facing fixation element 310' is offset from a central portion of the body 302 and the lengths L₁ and L₂ have such a ratio, the portion of the body 302 most distal from the interface-facing fixation element 310', for example, the portion of the body 302 nearer to the second edge 306, can provide for a better gripping region when attaching or detaching the fixation structure 300 to or from the patient's face.

In some embodiments, the fixation structure 300 has an increased area that can act as a bearing portion. For example, the fixation structure 300 can include a tab or other protrusion, such as on the first, second, third, and/or fourth edges 304, 306, 308A, 308B. The bearing portion can enable a user (e.g., a nurse) to engage (e.g., push down on) the bearing portion with a finger or instrument. This can aid in disconnecting the fixation structure 300 from the tubing (e.g., a nasogastric or nasojejunal tubes) and/or the patient interface. In certain variants, the bearing portion can aid in removing the tubing from the patient. In some embodiments, the bearing portion can help stabilize the fixation structure 300 and/or can reduce the disruption of the fixation structure 300 on the patient's face.

It should be understood that variations of the fixation structure 300 illustrated in Figure 13A are contemplated. For example, in some configurations, the concave curve might be present on only one of the third or fourth edges 308A, 308B, such as the edge that, in use, faces toward the patient's eye or eyelid. In some configurations, instead of having a concave curve, one of the third or fourth edges 308A, 308B might only extend farther from the centerline or center portion of the fixation structure 300 at a part of the third or fourth edges 308A, 308B proximal to one of the first and second edges 304, 306. In some configurations, portions of the third and fourth edges 308A, 308B proximal to the second edge 306 may project at lesser distance, or an approximately equal distance, from the centerline or center portion of the body 302 than portions of the third and fourth edges 308A, 308B proximal to the first edge 304. In some configurations, the concave curve may have a more exaggerated shape, including but not limited to a crescent shape. In some configurations, the interface-facing fixation element 310' may be present at or near the center of the fixation structure 300 rather than offset from the center. In some configurations, the interface-facing fixation element 310' may occupy most or substantially the entirety of the interface-facing region 301' of the fixation structure 300.

Figure 13B shows a view of the fixation structure 300 of Figure 13A along section C-C. As shown, the body 302 of the fixation structure 300 has a thickness t₁ and the interface-facing fixation element 310' has a thickness t₂. It has been realized that high flexibility or a low Young's modulus is desired for the body 302 to allow it to conform to changes in the shape of the face, due to, for example, crying or the expression of emotions. The thickness t₁ of the body 302 can be, for example, in the range of 0.05 - 0.35 mm, or 0.10 - 0.30 mm, or 0.15 - 0.25 mm In some embodiments, the thickness t₁ of the body 302 can be, for example, in the range of 0.05 - 5.0 mm ±10%, 0.05 - 3.0 mm ±10%, 0.1 - 1.0 mm ±10%, 0.178 - 0.76 mm ±10%, 0.178 - 0.381 mm ±10%, 0.38 - 0.76 mm ±10%, or approximately 0.76 mm ±10%. In some embodiments, the thickness t₁ of the body 302 is equal to or approximately 0.178 mm. In some embodiments, the thickness t₁ of the body 302 may be equal to, about equal to, or less than, the thickness t₂ of the interface-facing fixation element 310'. The body 302 having a relatively low thickness t₁ can improve the overall flexibility of the fixation structure 300. In some embodiments, the body 302 includes a material with a low Young's modulus. In certain variants, the entirety of the body 302 or substantially the entirety of the body 302 includes a material with a low Young's modulus. In some configurations, increasing the flexibility of the body 302 may cause the patient-facing region 301 of the body 302 to not adhere as strongly to the patient's face. Alternatively, increasing the adherence between the patient-facing region 301 of the body 302 and the patient's face can decrease the flexibility of the body 302. Thus, it is desirable to optimize the adherence between the patient-facing region 301 of the body 302 and the patient's face, while maintaining flexibility. Different materials may have different effects on flexibility and/or adherence of the body 302.

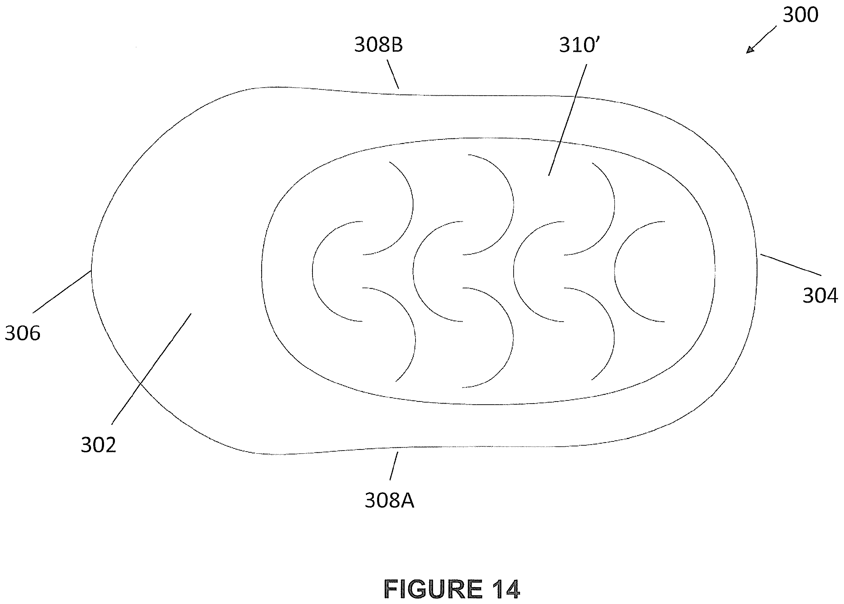

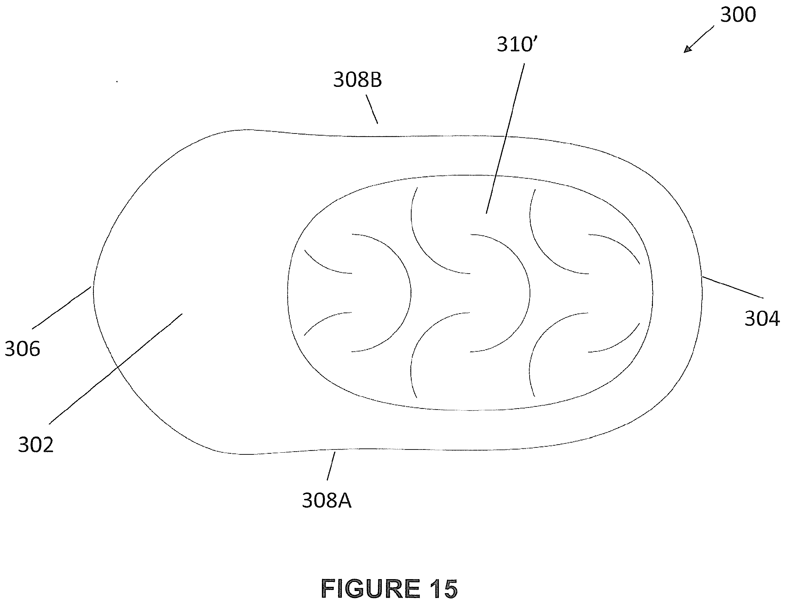

Figures 14 and 15 show interface-facing views of additional non-limiting embodiments for the fixation structure 300. The embodiments of Figure 14 and 15 can include any of the features of the embodiment of Figure 13A. For example, as illustrated, the embodiments of Figure 14 and 15 can include the body 302, the first edge 304, and the second edge 306. The first edge 304 can face toward the patient's nose or mouth in use and the second edge 306 can face away from the patient's nose or mouth in use. The opposed third and fourth edges 308A, 308B can extend between the first and second edges 304, 306. As shown, the third and fourth edges 308A, 308B can include concave curves (e.g., with respect to a longitudinal axis of the fixation structure 300).

The fixation structure 300 can include the interface-facing fixation element 310'. The interface-facing fixation element 310' is on the interface-facing region 301' of the fixation structure 300 and thus, faces away from the patient's face. The interface-facing fixation element 310' can be offset from a central portion of the body 302. In the illustrated configuration, the interface-facing fixation element 310' is positioned such that it is closer to the first edge 304 than to the second edge 306.

The interface-facing fixation element 310' can include one or more slits. The slits can be in a pattern including alternating columns of shapes of slits. For example, as illustrated, each column can include arched and/or semicircular slits with shapes that face the opposite direction to, and are at least partially offset from, the columns on either side. In some embodiments, portions of the columns of slits overlap in the longitudinal direction of the fixation structure 300.

In some cases, tears, mucus, vomit, or other liquids or liquid-solid mixtures can be present around the fixation structure 300 in use. The presence of such materials can reduce the adherence of the patient-facing region 301 of the fixation structure 300 to the patient. It has been realized that it is desirable to construct the body 302 of the fixation structure 300 from a material having a relatively low hygroscopicity to reduce the loss of adherence of the patient-facing region 301 to the face due to the presence of liquids or liquid-solid mixtures.

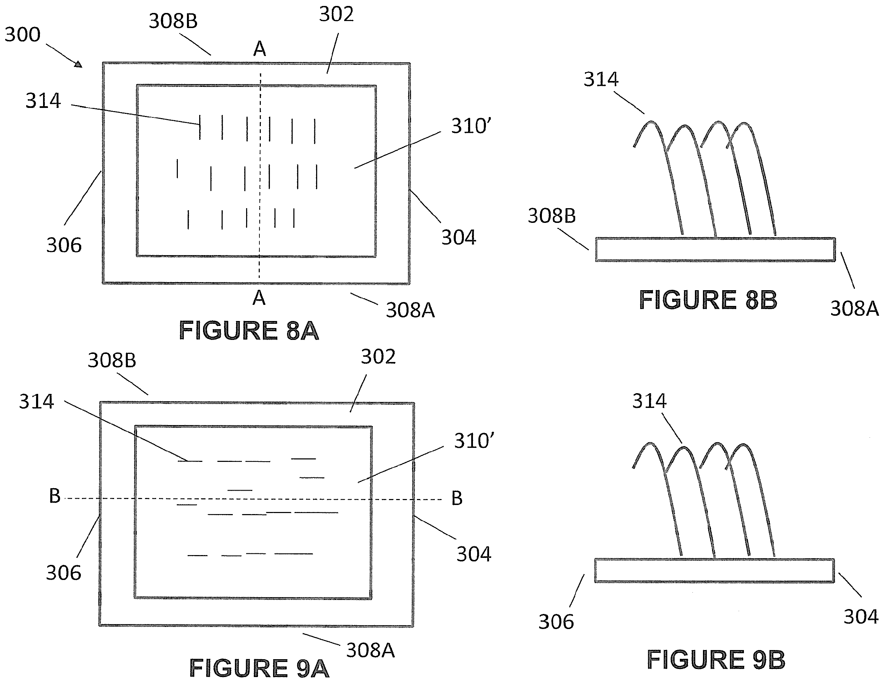

Figure 8A shows an exemplary non-limiting configuration for the fixation structure 300 including the interface-facing fixation element 310'. The interface-facing fixation element 310' includes a hooked pad that forms a 'hook-and-loop' style connection with the one of the attachment structures 216A, 216B of the patient interface 200. The hooked pad includes hooks 314. Figure 8B shows a close-up cross-section along dotted line A-A viewed from the first edge 304. As shown, the hooks 314 extend toward the fourth edge 308B of the body 302 of the fixation structure 300. In some configurations, the hooks 314 additionally or alternatively extend toward the third edge 308A of the body 302 of the fixation structure 300. In some cases, if the fixation structure 300 is used on the face of a patient such as an infant or neonate, the patient is inclined to attempt to remove the fixation structure 300 by grasping either the first or second edges 304, 306 and pulling the respective edge across the length of the fixation structure 300. It has been realized that the strength of the hook-and-loop securement is anisotropic relative to the orientation of the hooks 314.

In some configurations, and as seen in Figure 9A and Figure 9B, the hooks 314 may be arranged such that they extend toward the second edge 306. (Figure 9B is a cross-section of Figure 9A along line B-B viewed from the third edge 308A.) In other configurations the hooks 314 may be arranged such that they extend toward the first edge 304, the second edge 306, or the first and second edges 304, 306. If the hooks 314 are arranged such that they extend toward the first and/or second edges 304, 306, the strength of the hook-and-loop securement may be such that uncoupling is more greatly resisted when forces are applied along axes substantially parallel to the third and/or fourth edges 308A, 308B than when forces are applied along axes substantially parallel to the first and/or second edges 304, 306. As loops complementary to the hooks 314 would be urged substantially toward and/or against the length of the hooks 314 when such forces are applied rather than being urged in directions substantially perpendicular to the hooks 314, the forces are more greatly resisted. In some configurations, the hooks 314 may extend in multiple directions. In some such configurations, the hooks 314 may extend in, for example, substantially perpendicular directions to mitigate the anisotropicity of the strength of the hook-and-loop securement. In some configurations, the angle of the hooks 314 can vary to suit different applications. In some configurations, a combination of different angles can be used to determine the strength of securement between the interface-facing fixation element 310' and the patient interface 200. The hooks 314 can be positioned at a multiplicity of different angles and/or different directions, to aid with securement strength.

In an embodiment, a combination of at least one of the embodiments of the slits illustrated in Fig 5C, and Figs. 11A - 12H, and the configuration of the hooks 314 can be used to maximize securement strength and/or flexibility of the interface-facing fixation element 310' with the patient interface 200.

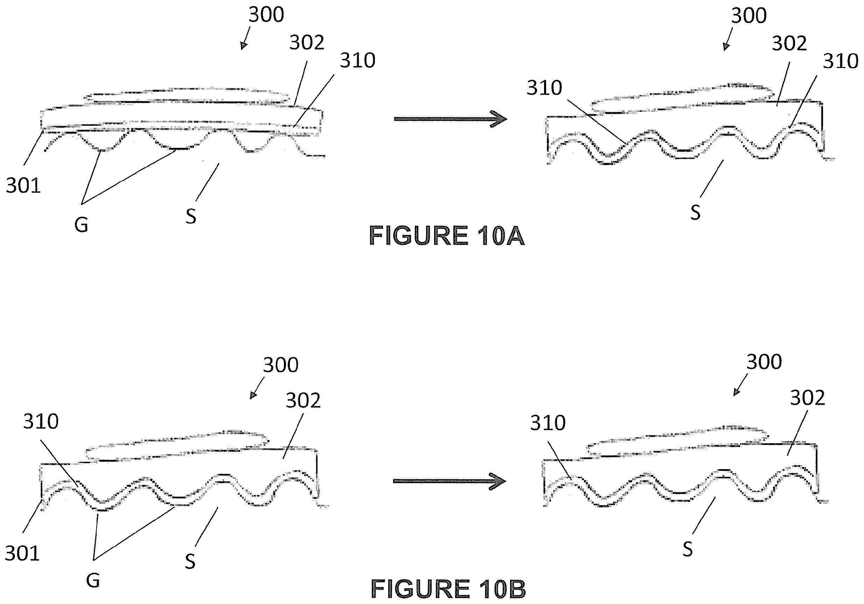

Figure 10A shows the use of the fixation structure 300 on a skin surface S of the patient's face. The patient-facing region 301 includes the patient-facing fixation element 310, which can be constructed from a material that adheres to the skin surface S, for example, a hydrocolloid-based adhesive. As shown, on a microscopic level the skin surface S includes an irregular thickness along the skin surface S and includes a number of bumps and gaps G. Upon application to the skin surface S, the patient-facing fixation element 310 initially adheres to only a portion of the skin surface S (see left drawing of Figure 10A). Over the course of a period of time t, the adhesive patient-facing fixation element 310 flows into the gaps G and increases in adhesion (see right drawing of Figure 10A). However, it has been realized that discomfort is experienced when the fixation structure 300 is removed from the skin surface S, because, in some cases, hair and part of the stratum corneum of the skin surface S is simultaneously removed. Additionally, in some cases it is difficult to predict the strength of the adhesive at times after the application of the adhesive to the skin surface S.

Figure 10B shows the use of an embodiment of the fixation structure 300, wherein the patient-facing fixation element 310 is constructed from a silicone-based adhesive. As shown, on a microscopic level, upon application to the skin surface, the patient-facing fixation element 310 rapidly flows into the gaps G and quickly reaches a maximum adherence to the skin. Accordingly, the strength of the adhesive at times after the application of the adhesive becomes more predictable. The silicone-based adhesive is less adhesive than the hydrocolloid-based adhesive, and requires less force to remove than the hydrocolloid-based adhesive. Increasing the contact area between the adhesive and the skin can allow sufficient adhesion there between, and reduce the removal of part of the skin surface when the fixation structure 300 is removed. If the patient-facing fixation element 310 is constructed from a silicone-based adhesive, then the discomfort of removal or detachment of the fixation structure 300 from the skin can be reduced.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to."

Where, in the foregoing description reference has been made to integers or components having known equivalents thereof, those integers or components are herein incorporated as if individually set forth.

The disclosed methods, apparatus and systems may also be said broadly to comprise the parts, elements and features referred to or indicated in the disclosure, individually or collectively, in any or all combinations of two or more of said parts, elements or features. Further, this disclosure includes combinations of the various features, aspects, methods, properties, characteristics, qualities, attributes, elements, and the like of the various embodiments. For example, any particular feature, aspect, method, property, characteristic, quality, attribute, element, or the like in connection with an embodiment herein can be used in connection with any other embodiment herein.

Reference to any prior art in this specification is not, and should not be taken as, an acknowledgement or any form of suggestion that that prior art forms part of the common general knowledge in the field of endeavour in any country in the world.

Language of degree used herein, such as the terms "approximately," "about," "generally," and "substantially," as used herein represent a value, amount or characteristic close to the stated value, amount or characteristic that still performs a desired function or achieves a desired result. For example, the terms "generally parallel" and "substantially parallel" refer to a value, amount or characteristic that can depart from exactly parallel by less than or equal to 15 degrees, 10 degrees, 5 degrees, 3 degrees, 1 degree, 0.1 degree, or otherwise.

Some embodiments have been described in connection with the accompanying figures. The figures are drawn to scale, but such scale should not be limiting. Dimensions and proportions other than what are shown are contemplated and are within the scope of this disclosure. Distances, angles, shapes, etc. are merely illustrative and do not necessarily bear an exact relationship to actual dimensions and layout of the devices illustrated. Components can be added, removed, and/or rearranged.

Although the present disclosure has been described in terms of certain embodiments, other embodiments apparent to those of ordinary skill in the art also are within the scope of this disclosure.

## Claims

1. A fixation structure (300) for securing a patient interface (200) on a patient's face, the fixation structure (300) comprising:
a body (302);
wherein a first side of the body (302) facing away from the patient's face and toward the patient interface (200) comprises a fixation element (310'), the fixation element attachable to a complementary fastener of an attachment structure (216A, 216B) of the patient interface (200); and
**characterized in that** the fixation element (310') has a pattern of slits which facilitate flexibility to the fixation element (310'),
wherein the pattern of slits includes alternating columns of shapes of slits.

2. The fixation structure (300) of claim 1, wherein each of the columns includes arched and/or semicircular slits.

3. The fixation structure (300) of claim 2, wherein the slits in each of the columns have shapes that face the opposite direction to, and are at least partially offset from, the columns on either side.

4. The fixation structure (300) of any of claims 1 to 3, wherein the body (302) comprises:
a first edge (304);
a second edge (306) opposing the first edge (304), such that in use on the patient's face the second edge (306) is farther away than the first edge (304) from the patient's nose or mouth;
opposing third and fourth edges (308A, 308B) extending between the first and second edges (304, 306),
wherein the first side extends between the first and second edges (304, 306).

5. The fixation structure (300) of claim 4, wherein at least some of the slits extend to at least one of the first, second, third, or fourth edges (304, 306, 308A, 308B).

6. The fixation structure (300) of claim 4, wherein at least some of the slits is arranged in columns of slits that extend to the third and fourth edges in alternation.

7. The fixation structure (300) of claim 2, wherein the semicircular slits overlap in the longitudinal direction of the fixation structure (300).

8. The fixation structure (300) of claim 6, wherein portions of the columns of slits overlap in the longitudinal direction of the fixation structure (300).

9. The fixation structure (300) of claim 6 or 8, wherein each of the columns of slits comprises slits with semicircular or crescent shapes that face the opposite direction to, and are at least partially offset toward the third or fourth edge from, semicircular or crescent shapes of slits in an adjacent column.

10. The fixation structure (300) of any of claims 4 to 9, wherein the fixation element is positioned closer to the first edge than to the second edge.

11. The fixation structure (300) of any of claims 4 to 10, wherein at least some of the slits extend toward the third and fourth edges.

12. The fixation structure (300) of any of claims 4 to 11, wherein at least some of the slits that are closer to the first and second edges are shorter than at least some of the slits that are farther from the first and second edges.

13. The fixation structure (300) of any of claims 4 to 12, wherein at least some of the slits do not extend across a centerline or central portion midway between the third and fourth edges.

14. The fixation structure (300) of any of claims 4 to 13, further comprising a second side extending between the first and second edges, the second side facing toward the patient's face and away from the patient interface in use, the second side comprising a second fastener that is attachable to the patient's face.

## Patentansprüche

1. Fixierungsstruktur (300) zum Fixieren einer Patientenschnittstelle (200) am Gesicht eines Patienten, wobei die Fixierungsstruktur (300) Folgendes umfasst:
einen Körper (302);
wobei eine von dem Gesicht des Patienten weg und in Richtung der Patientenschnittstelle (200) weisende erste Seite des Körpers (302) ein Fixierungselement (310') umfasst, wobei das Fixierungselement an einem komplementären Befestigungselement einer Befestigungsstruktur (216A, 216B) der Patientenschnittstelle (200) befestigt werden kann; und
**dadurch gekennzeichnet, dass** das Fixierungselement (310') ein Muster von Schlitzen aufweist, die die Biegsamkeit des Fixierungselement (310') fördern,
wobei das Muster von Schlitzen abwechselnde Spalten von Formen von Schlitzen umfasst.

2. Fixierungsstruktur (300) nach Anspruch 1, wobei die Spalten jeweils bogenförmige und/oder halbkreisförmige Schlitze umfassen.

3. Fixierungsstruktur (300) nach Anspruch 2, wobei die Schlitze in jeder der Spalten Formen aufweisen, die in die entgegengesetzte Richtung der Spalten auf beiden Seiten weisen und mindestens teilweise von diesen versetzt sind.

4. Fixierungsstruktur (300) nach einem der Ansprüche 1 bis 3, wobei der Körper (302) Folgendes umfasst:
eine erste Kante (304);
eine zweite Kante (306), die der ersten Kante (304) gegenüberliegt, sodass im Einsatz am Gesicht des Patienten die zweite Kante (306) weiter von der Nase oder dem Mund des Patienten entfernt ist als die erste Kante (304);
eine dritte und eine gegenüberliegende vierte Kante (308A, 308B), die sich zwischen der ersten und der zweiten Kante (304, 306) erstrecken,
wobei sich die erste Seite zwischen der ersten und der zweiten Kante (304, 306) erstreckt.

5. Fixierungsstruktur (300) nach Anspruch 4, wobei sich mindestens einige der Schlitze zu mindestens einer von der ersten, der zweiten, der dritten oder der vierten Kante (304, 306, 308A, 308B) erstrecken.

6. Fixierungsstruktur (300) nach Anspruch 4, wobei mindestens einige der Schlitze in Spalten von Schlitzen angeordnet sind, die sich abwechselnd zu der dritten und der vierten Kante erstrecken.

7. Fixierungsstruktur (300) nach Anspruch 2, wobei die halbkreisförmigen Schlitze einander in der Längsrichtung der Fixierungsstruktur (300) überlagern.

8. Fixierungsstruktur (300) nach Anspruch 6, wobei Abschnitte der Spalten von Schlitzen einander in der Längsrichtung der Fixierungsstruktur (300) überlagern.

9. Fixierungsstruktur (300) nach Anspruch 6 oder 8, wobei die Spalten von Schlitzen jeweils Schlitze mit Halbkreis- oder Sichelformen umfassen, die in die entgegengesetzte Richtung weisen wie Halbkreis- oder Sichelformen von Schlitzen in einer benachbarten Spalte und von diesen mindestens teilweise in Richtung der dritten oder der vierten Kante versetzt sind.

10. Fixierungsstruktur (300) nach einem der Ansprüche 4 bis 9, wobei das Fixierungselement näher an der ersten Kante als an der zweiten Kante positioniert ist.

11. Fixierungsstruktur (300) nach einem der Ansprüche 4 bis 10, wobei sich mindestens einige der Schlitze in Richtung der dritten und der vierten Kante erstrecken.

12. Fixierungsstruktur (300) nach einem der Ansprüche 4 bis 11, wobei mindestens einige der Schlitze, die näher bei der ersten und der zweiten Kante liegen, kürzer sind als mindestens einige der Schlitze, die weiter von der ersten und der zweiten Kante entfernt sind.

13. Fixierungsstruktur (300) nach einem der Ansprüche 4 bis 12, wobei sich mindestens einige der Schlitze nicht über eine Mittellinie oder einen mittleren Abschnitt auf halber Strecke zwischen der dritten und der vierten Kante erstrecken.

14. Fixierungsstruktur (300) nach einem der Ansprüche 4 bis 13, ferner umfassend eine zweite Seite, die sich zwischen der ersten und der zweiten Kante erstreckt, wobei die zweite Seite im Einsatz in Richtung des Gesichts des Patienten und von der Patientenschnittstelle weg weist, wobei die zweite Seite ein zweites Befestigungselement umfasst, das am Gesicht des Patienten befestigt werden kann.

## Revendications

1. Structure de fixation (300) destinée à fixer une interface patient (200) sur le visage d'un patient, la structure de fixation (300) comprenant :
un corps (302) ;
dans laquelle un premier côté du corps (302) orienté à l'opposé du visage du patient et vers l'interface patient (200) comprend un élément de fixation (310'), l'élément de fixation pouvant être attaché à une attache complémentaire d'une structure d'attache (216A, 216B) de l'interface patient (200) ; et
**caractérisée en ce que** l'élément de fixation (310') a un motif de fentes qui facilitent la flexibilité de l'élément de fixation (310'),
dans laquelle le motif de fentes inclut des colonnes alternées de formes de fentes.

2. Structure de fixation (300) selon la revendication 1, dans laquelle chacune des colonnes inclut des fentes arquées et/ou semi-circulaires.

3. Structure de fixation (300) selon la revendication 2, dans laquelle les fentes dans chacune des colonnes ont des formes qui sont orientées vers la direction opposée aux colonnes sur chaque côté, et sont au moins partiellement décalées par rapport à celles-ci.

4. Structure de fixation (300) selon l'une quelconque des revendications 1 à 3, dans laquelle le corps (302) comprend :
un premier bord (304) ;
un deuxième bord (306) opposé au premier bord (304), de sorte que pendant l'utilisation sur le visage du patient le deuxième bord (306) est plus éloigné que le premier bord (304) du nez ou de la bouche du patient ;
des troisième et quatrième bords opposés (308A, 308B) s'étendant entre les premier et deuxième bords (304, 306),
dans laquelle le premier côté s'étend entre les premier et deuxième bords (304, 306).

5. Structure de fixation (300) selon la revendication 4, dans laquelle au moins certaines des fentes s'étendent vers au moins l'un des premier, deuxième, troisième, ou quatrième bords (304, 306, 308A, 308B).

6. Structure de fixation (300) selon la revendication 4, dans laquelle au moins certaines des fentes sont agencées en colonnes de fentes qui s'étendent vers les troisième et quatrième bords en alternance.

7. Structure de fixation (300) selon la revendication 2, dans laquelle les fentes semi-circulaires se chevauchent dans la direction longitudinale de la structure de fixation (300).

8. Structure de fixation (300) selon la revendication 6, dans laquelle des parties des colonnes de fentes se chevauchent dans la direction longitudinale de la structure de fixation (300).

9. Structure de fixation (300) selon la revendication 6 ou 8, dans laquelle chacune des colonnes de fentes comprend des fentes ayant des formes semi-circulaires ou de croissant qui sont orientées vers la direction opposée aux formes semi-circulaires ou de croissant de fentes dans une colonne adjacente, et sont au moins partiellement décalées vers le troisième ou quatrième bord par rapport aux formes semi-circulaires ou de croissant de fentes dans une colonne adjacente.

10. Structure de fixation (300) selon l'une quelconque des revendications 4 à 9, dans laquelle l'élément de fixation est positionné plus proche du premier bord que du deuxième bord.

11. Structure de fixation (300) selon l'une quelconque des revendications 4 à 10, dans laquelle au moins certaines des fentes s'étendent vers les troisième et quatrième bords.

12. Structure de fixation (300) selon l'une quelconque des revendications 4 à 11, dans laquelle au moins certaines des fentes qui sont plus proches des premier et deuxième bords sont plus courtes qu'au moins certaines des fentes qui sont plus éloignées des premier et deuxième bords.

13. Structure de fixation (300) selon l'une quelconque des revendications 4 à 12, dans laquelle au moins certaines des fentes ne s'étendent pas sur une ligne centrale ou une partie centrale à mi-chemin entre les troisième et quatrième bords.

14. Structure de fixation (300) selon l'une quelconque des revendications 4 à 13, comprenant en outre un deuxième côté s'étendant entre les premier et deuxième bords, le deuxième côté orienté vers le visage du patient et à l'écart de l'interface patient pendant l'utilisation, le deuxième côté comprenant une deuxième attache qui peut être attachée au visage du patient.
